# EUROPEAN PATENT SPECIFICATION

(11) **EP 1 877 757 B1**
(45) Date of publication and mention of the grant of the patent: **10.03.2021**
(21) Application number: 06751406.7
(22) Date of filing: 26.04.2006
(51) Int. Cl.: G01N 21/76, G01N 21/64, G01N 33/18, G01N 21/77

(54) **METHOD FOR USING AN ALL SOLID-STATE FLUOROMETER IN MONITORING AND CONTROLLING CHEMICALS IN WATER**
VERFAHREN ZUR VERWENDUNG EINER FESTKÖRPER-FLUOROMETERS ZUR ÜBERWACHUNG UND STEUERUNG VON CHEMIKALIEN IN WASSER
PROCEDE D'UTILISATION D'UN FLUOROMETRE ENTIEREMENT TRANSISTORISE DANS LA SURVEILLANCE ET LE CONTROLE DES SUBSTANCES CHIMIQUES PRESENTES DANS DE L'EAU

(30) Priority: 02.05.2005 US 119970
(43) Date of publication of application: 16.01.2008
(73) Proprietor: Nalco Company, Naperville, IL 60563-1198 (US)
(72) Inventor: WETEGROVE, Robert, L., Winfield, Illinois 60190 (US); BANKS, Rodney, H., Aurora, Illinois 60504 (US)
(74) Representative: Godemeyer Blum Lenze Patentanwälte Partnerschaft mbB - werkpatent
(86) International application number: PCT/US2006/015677
(87) International publication number: WO 2006/118876

(56) References cited:
- WO-A1-01/49876
- WO-A1-01/81654
- WO-A1-01/84125
- WO-A1-98/57153
- WO-A1-03/082447
- JP-A- 2003 075 348
- JP-A- 2004 157 018
- US-A- 5 435 969
- US-A- 5 905 570
- US-A1- 2003 098 421
- US-A1- 2005 025 660
- US-A1- 2005 247 114
- US-B1- 6 255 118
- US-B1- 6 255 118
- ZA-B- 9 804 976
- C D Mcguinness ET AL: "A new sub-nanosecond LED at 280 nm: application to protein fluorescence", MEASUREMENT SCIENCE AND TECHNOLOGY., vol. 15, no. 11, 12 October 2004 (2004-10-12), pages 19-22, XP055570081, GB ISSN: 0957-0233, DOI: 10.1088/0957-0233/15/11/L02

## Description

### FIELD OF THE INVENTION

The present invention generally relates to the use of a solid-state fluorometer in a method for monitoring and controlling the concentration of chemicals added to and present in water systems.

### BACKGROUND OF THE INVENTION

U.S. Patent No. 6,255,118 issued to Alfano et al. describes a method for monitoring and control of the concentration of chemicals in industrial systems by utilizing a solid-state fluorometer with an excitation source that is either a light emitting diode or a solid state diode laser and using said solid-state fluorometer to determine the concentration of a fluorescent tracer that is added to the industrial system in a known proportion to the chemical added to the industrial system. McGuinness et al. (Meas. Sci. Tech. 15, 2004, L19-L22) discloses a method for exciting and recording the intrinsic fluorescence of proteins. US 2003/098421 A1 discloses a process for detecting NADP (nicotinamide adenine dinucleotide phosphate) and BG spores in aqueous media. The excitation wavelength of NADH is centered at 340 nm in the near ultraviolet spectrum,

### SUMMARY OF THE INVENTION

The present invention provides for a method for monitoring the concentration of one or more chemicals added to a water system.

The present invention is directed to a method for fluorometric monitoring of one or more biological materials in a water system that utilize a solid state fluorometer that has one or more excitation sources that are either a light emitting diode or a solid state laser. The fluorometer has one or more detectors that receive fluorescence from the excitation of the water system and produce an output signal proportional to the quantity of fluorescence received by the detectors. The fluorometer as described above is used to detect the fluorescence of biological materials that are in the water system. The fluorometer is programmed to produce an output signal proportional to the detected fluorescence. Optionally, controlling the concentration of the biological materials in the water system is based on the output signal from said fluorescent biological materials detected by the fluorometer. The method of the present invention is defined in claim 1.

Additional features and advantages of the present invention are described in, and will be apparent from, the detailed description of the presently preferred embodiments.

### DESCRIPTION OF THE PREFERRED EMBODIMENTS

Throughout this patent application the following terms have the indicated meanings: "About" means nearly or equal to.

"Water system" means a water system for consumer or industrial applications.

"NDSA" means naphthalene disulfonic acid.

"BSA" means bovine serum albumin.

"EWL" means egg white lysozyme.

"NADH" means nicotinamide adenine dinucleotide.

"NADPH" means nicotinamide adenine dinucleotide phosphate.

"LED" means light emitting diode.

"Biological materials" means living organisms or materials derived from living organisms.

"Chemical treatment" means a protocol invoking the addition of chemicals to a water system to produce a desired effect.

The solid-state diode laser or light emitting diode fluorometer instrument in the method of the present invention is suitable for use in several industrial and consumer water applications. These include, but are not limited to, cooling water systems, boiler water systems, pulp slurries, ceramic slurries, waste-treatment, mining, agriculture, oil-field applications, drinking or potable water supplies, a reverse osmosis system, commercial and consumer hot water supplies and equipment, swimming pools and spas, amusement park rides, food processing and decorative fountains.

In one embodiment, the solid state fluorometer detector is a silicon photodiode. In another embodiment, the solid state fluorometer is a photomultiplier tube.

In another embodiment, the solid state fluorometer is used to monitor biological materials in a water system.

In yet another embodiment, the biological materials are selected from the group consisting of: amino acids; NADH; nucleic acids; tryptophan, tyrosine; adenine triphosphate; calcium dipicolinate; NAD(P)H; flavins; 3,4 dihydroxyphenyalanine; kynurenine; Serotonin; phenylalanine; dopamine; histamine; Vitamin A; Vitamin B12; estrogen; adenine diphosphate; adenine; adenosine; bovine serum albumin; egg white lysozyme; microorganisms; toxins; spores; viruses; algae; fungi; and proteins.

For example, a 280 nm LED fluorometrically detects tryptophan, a common amino acid in proteins produced by living entities, the 340 nm LED detects NAD(P)H/NAD(P)⁺ ratios associated with cellular metabolism, and scattering can detect film deposit and biofilm formation.

According to the present invention, a chemical treatment is applied to a water system in response to the output signal from said chemical detected by said fluorometer. The chemical treatment is a biocidal, biostatic, or other microorganism control agent. Both biocidal and biostatic control agents, include, but are not limited to the following compounds: hypohalous acids; halogen release compounds; halosulfamates; chlorine dioxide; ozone; peroxygen compounds; dibromonitrilopropionamide; isothiazolins; quaternary compounds, glutaraldehyde; triazines; and surfactants such as ethylene oxide / propylene oxide copolymers and polyalkylglycosides.

In another embodiment, the effective amount of chemical treatment is added to said water system to prevent microbial or contamination in said water system.

The biological materials fluoresce at an excitation wavelength from about 260 nm to about 350 nm.

Besides biological materials, other chemicals can be monitored such as fluorescent tracers used in water treatment. Some polymers and other chemical actives exhibit natural fluorescence in the region that solid-state LEDs or laser diodes emit. Devices of this invention can be made to measure DAXAD polymer or naphthalene sulfonic acid/formaldehyde sodium salt copolymer; NexGuard polymer or acrylic acid/styrene sulfonate copolymer and its lower molecular weight decomposition byproducts as well as naphthalene disulfonic acid, benzotriazole, tolyltriazole, hydroquinone, gallic acid, pyrogallol, sulfonated anthracenes, and fluorescently tagged polymer(s) which may be in the form of a concentration indicator (U.S. patent no. 5,435,969), or tagged polymers (U.S. patent nos. 5,171,450 and 6,645,428).

The following examples are presented to describe preferred embodiment and utilization of the invention and are not meant to limit the invention unless otherwise stated in the claims appended hereto.

### EXAMPLES

### Example 1:

Tryptophan is an amino acid that is one of the fluorescent components of proteins. Since proteins are essential elements of living things it is useful to be able to detect such fluorescent species if one is interested in detecting living organisms or residual protein contamination caused by living organisms. The following table shows the response of an LED-based detector to various low levels of the amino acid tryptophan.

| Table One | |
|---|---|
| Tryptophan Fluorescence with UV LED | |
| Tryptophan µg/L | Fluorescence with 280nm Excitation |
| 0 | 22.6 |
| 10 | 34.65 |
| 100 | 151.3 |

### Example 2:

One example of a protein containing fluorescent amino acids is bovine serum albumin (BSA), a component of cow's blood. The next table shows the proportional response to BSA from an LED-based fluorescence detector. This shows useful detection of protein that could be a component of biological fouling in an industrial system or for detecting protein contamination in meat processing equipment.

| Table Two | |
|---|---|
| Bovine Serum Albumin Fluorescence with UV LED | |
| Bovine Serum Albumin (mg/L) | Fluorescence with 280nm Excitation |
| 0 | 20.35 |
| 1 | 21 |
| 10 | 33.2 |
| 100 | 159.4 |

### Example 3:

Another example of a protein containing fluorescent amino acids is egg white lysozyme, a component of hen's eggs. The next table shows detection of EWL with an LED-based fluorescence detector. This device could be used for detecting protein contamination in food processing or from microorganisms.

| Table Three | |
|---|---|
| Egg White Lysozyme Fluorescence with UV LED | |
| Egg White Lysozyme mg/L | Fluorescence with 280nm Excitation |
| 0 | 19.6 |
| 1 | 20.53 |
| 10 | 40.6 |
| 100 | 176.3 |

### Example 4:

A component of living organisms is nicotinamide adenine dinucleotide. Known as NADH, this substance participates in chemical reduction and oxidation reactions in cells and is present in all living things, but is degraded rapidly after death. It is therefore useful to be able to detect NADH as an indicator of the presence of living organisms in biological contamination. The next chart shows detection of NADH by an LED detector with fluorescence excitation at 340nm.

| Table Four | |
|---|---|
| NADH Fluorescence with UV LED | |
| NADH µmoles/L | Fluorescence with 340nm Excitation |
| 0 | 0 |
| 5 | 30 |
| 25 | 157 |
| 50 | 262 |

### Example 5:

The following table and chart show use of the LED fluorescence detection system to examine a series of samples containing levels of living *Pseudomonas aeruginosa* bacteria. These data show a threshold of fluorescence detection of about one thousand bacteria per mL at excitation wavelengths of 280nm and 340nm.

| Table Five | | |
|---|---|---|
| Living bacteria count (CFU) and fluorescence at 280nm and 340nm | | |
| log10 cfu/ml | Fluorescence with 280nm Excitation (mV) | Fluorescence with 340nm Excitation (mV) |
| 1.60E+01 | 21.3 | 8.5 |
| 1.60E+02 | 21.3 | 12.5 |
| 1.60E+03 | 24.2 | 60 |
| 1.61E+04 | 43.5 | 477 |

### Reference example 6:

The following table shows data for fluorescence of NDSA using the 280 nm LED.

| Table Six | |
|---|---|
| NDSA | |
| NDSA (ppb) | Fluorescence with 280nm Excitation (mV) |
| 0 | 140 |
| 250 | 295 |
| 500 | 460 |
| 700 | 580 |

## Claims

1. A method for monitoring concentration of one or more chemicals in a water system, the method comprising the steps of:
a) providing a solid state fluorometer, wherein said fluorometer comprises:
i) one or more solid-state excitation sources to direct light in a specified direction, wherein said excitation source is a light emitting diode or a solid state laser diode,
ii) one or more detectors receiving the fluorescence from the excitation of the sample and producing an output signal proportional to the quantity of fluorescence received on the detector;
b) providing a water system;
c) using said fluorometer to detect the fluorescence of said chemicals in said water system;
d) programming said fluorometer to produce an output signal proportional to the detected fluorescence,
wherein said light emitting diode or a solid state laser diode emits light having a wavelength from about 260 nm to about 350 nm; and
wherein said chemicals are biological materials and selected from the group consisting of: amino acids; NADH; nucleic acids; tryptophan, tyrosine; adenine triphosphate; calcium dipicolinate; NAD(P)H; flavins; 3,4-dihydroxyphenylalanine; kynurenine; Serotonin; phenylalanine; dopamine; histamine; Vitamin A; Vitamin B 12; estrogen; adenine diphosphate; adenine; adenosine; bovine serum albumin; egg white lysozyme; microorganisms; toxins; spores; viruses; algae; fungi; and proteins; and
further comprising the step of providing an effective amount of chemical treatment to said water system in response to the output signal from said chemical detected by said fluorometer, where said chemical treatment is selected from the group consisting of: a biocidal control agent, a biostatic control agent, and microorganism control agent.

2. The method of claim 1 further comprising the step of controlling concentration of said chemicals in said water system based on said output signal from said fluorescent chemicals detected by said fluorometer.

3. The method of claim 1 wherein said detector is a silicon photodiode.

4. The method of claim 1 wherein said detector is a photomultiplier tube.

5. The method of claim 1 wherein said effective amount of chemical treatment is added to said water system to prevent microbial or contamination in said water system.

6. The method of claim 1 wherein said biocidal control agents and biostatic control agents are selected from the group consisting of: hypohalous acids; halogen release compounds; halosulfamates; chlorine dioxide; ozone; peroxygen compounds; dibromonitrilopropionamide; isothiazolins; quaternary compounds, glutaraldehyde; triazines; surfactants, ethylene oxide / propylene oxide copolymers; and polyalkylglycosides.

## Patentansprüche

1. Verfahren zum Überwachen einer Konzentration einer oder mehrerer Chemikalien in einem Wassersystem, wobei das Verfahren die folgenden Schritte umfasst:
a) Bereitstellen eines Festkörperfluorometers, wobei das Fluorometer Folgendes umfasst:
i) eine oder mehrere Festkörperanregungsquellen, um Licht in eine bestimmte Richtung zu lenken, wobei die Anregungsquelle eine Leuchtdiode oder eine Festkörperlaserdiode ist,
ii) einen oder mehrere Detektoren, die die Fluoreszenz von der Anregung der Probe empfangen und ein Ausgangssignal erzeugen, das proportional zu der auf dem Detektor empfangenen Fluoreszenzmenge ist;
b) Bereitstellen eines Wassersystems;
c) Verwenden des Fluorometers, um die Fluoreszenz der Chemikalien in dem Wassersystem zu detektieren;
d) Programmieren des Fluorometers, um ein Ausgangssignal zu erzeugen, das proportional zu der detektierten Fluoreszenz ist,
wobei die Leuchtdiode oder eine Festkörperlaserdiode Licht emittiert, das eine Wellenlänge von etwa 260 nm bis etwa 350 nm aufweist; und
wobei die Chemikalien biologische Materialien und aus der Gruppe ausgewählt sind, die aus Folgendem besteht:
Aminosäuren;
NADH;
Nukleinsäuren;
Tryptophan, Tyrosin;
Adenintriphosphat;
Calciumdipicolinat;
NAD(P)H;
Flavine;
3,4-Dihydroxyphenylalanin;
Kynurenin;
Serotonin;
Phenylalanin;
Dopamin;
Histamin;
Vitamin A;
Vitamin B 12;
Östrogen;
Adenindiphosphat;
Adenin;
Adenosin;
Rinderserumalbumin;
Eiweißlysozym;
Mikroorganismen;
Toxine;
Sporen;
Viren;
Algen;
Pilze; und
Proteine; und
ferner umfassend den Schritt eines Bereitstellens einer wirksamen Menge an chemischer Behandlung an das Wassersystem als Reaktion auf das Ausgangssignal von der durch das Fluorometer detektierten Chemikalie, wo die chemische Behandlung aus der Gruppe ausgewählt ist, die aus Folgendem besteht:
einem bioziden Kontrollmittel, einem biostatischen Kontrollmittel und einem Mikroorganismenkontrollmittel.

2. Verfahren nach Anspruch 1, ferner umfassend den Schritt eines Regulierens der Konzentration der Chemikalien in dem Wassersystem basierend auf dem Ausgangssignal von der durch das Fluorometer detektierten Chemikalie.

3. Verfahren nach Anspruch 1, wobei der Detektor eine Silizium-Fotodiode ist.

4. Verfahren nach Anspruch 1, wobei der Detektor ein Fotovervielfacher ist.

5. Verfahren nach Anspruch 1, wobei die wirksame Menge an chemischer Behandlung dem Wassersystem zugesetzt wird, um Mikroben oder eine Kontamination in dem Wassersystem zu verhindern.

6. Verfahren nach Anspruch 1, wobei die bioziden Regulationsmittel und die biostatischen Regulationsmittel aus der Gruppe ausgewählt sind, die aus Folgendem besteht:
unterhalogene Säuren;
Halogenfreisetzungsverbindungen;
Halosulfamaten;
Chlordioxid;
Ozon;
Peroxygenverbindungen;
Dibromonitrilopropionamid;
Isothiazolinen;
quaternären Verbindungen, Glutaraldehyd;
Triazinen;
Tensiden, Ethylenoxid/Propylenoxid-Copolymeren; und
Polyalkylglycosiden.

## Revendications

1. Procédé pour surveiller la concentration d'un ou de plusieurs produits chimiques dans un réseau d'alimentation en eau, le procédé comprenant les étapes :
a) de fourniture d'un fluorimètre à l'état solide, ledit fluorimètre comprenant :
i) une ou plusieurs sources d'excitation à semi-conducteurs pour diriger la lumière dans une direction spécifiée, ladite source d'excitation étant une diode électroluminescente ou une diode laser à semi-conducteurs,
ii) un ou plusieurs détecteurs recevant la fluorescence de l'excitation de l'échantillon et produisant un signal de sortie proportionnel à la quantité de fluorescence reçue sur le détecteur ;
b) de fourniture d'un réseau d'alimentation en eau ;
c) d'utilisation dudit fluorimètre pour détecter la fluorescence desdits produits chimiques dans ledit réseau d'alimentation en eau ;
d) de programmation dudit fluorimètre pour produire un signal de sortie proportionnel à la fluorescence détectée,
ladite diode électroluminescente ou une diode laser à semi-conducteurs émettant une lumière ayant une longueur d'onde d'environ 260 nm à environ 350 nm ; et
lesdits produits chimiques étant des matériaux biologiques et choisis dans le groupe constitué : d'acides aminés ; de NADH ; d'acides nucléiques ; de tryptophane, de tyrosine ; d'adénine triphosphate ; de dipicolinate de calcium ; de NAD(P)H ; de flavines ; de 3,4-dihydroxyphénylalanine; de kynurénine; de sérotonine ; de phénylalanine ; de dopamine ; d'histamine ; de vitamine A; de vitamine B12 ; d'oestrogène ; d'adénine diphosphate ; d'adénine ; d'adénosine ; d'albumine de sérum bovin ; de lysozyme de blanc d'œuf ; de micro-organismes ; de toxines ; de spores ; de virus ; d'algues ; de champignons ; et de protéines ; et
comprenant en outre l'étape de fourniture d'une quantité efficace de traitement chimique audit réseau d'alimentation en eau en réponse au signal de sortie dudit produit chimique détecté par ledit fluorimètre, ledit traitement chimique étant choisi dans le groupe constitué : d'un agent de contrôle biocide, d'un agent de contrôle biostatique et d'un agent de contrôle des micro-organismes.

2. Procédé selon la revendication 1, comprenant en outre l'étape de contrôle de la concentration desdits produits chimiques dans ledit réseau d'alimentation en eau sur la base dudit signal de sortie desdits produits chimiques fluorescents détectés par ledit fluorimètre.

3. Procédé selon la revendication 1, dans lequel ledit détecteur est une photodiode au silicium.

4. Procédé selon la revendication 1, dans lequel ledit détecteur est un tube photomultiplicateur.

5. Procédé selon la revendication 1, dans lequel ladite quantité efficace de traitement chimique est ajoutée audit réseau d'alimentation en eau pour empêcher des microbes ou une contamination dans ledit réseau d'alimentation en eau.

6. Procédé selon la revendication 1, dans lequel lesdits agents de contrôle biocides et agents de contrôle biostatiques sont choisis dans le groupe constitué : d'acides hypohalogéneux ; de composés de libération d'halogène ; d'halosulfamates ; de dioxyde de chlore ; d'ozone ; de composés peroxygénés ; de dibromonitrilopropionamide; d'isothiazolines ; de composés quaternaires, de glutaraldéhyde; de triazines ; de tensioactifs, de copolymères d'oxyde d'éthylène / d'oxyde de propylène ; et de polyalkylglycosides.
